# EUROPÄISCHE PATENTSCHRIFT

(11) **EP 1 060 154 B1**
(45) Veröffentlichungstag und Bekanntmachung des Hinweises auf die Patenterteilung: **29.01.2003**
(21) Anmeldenummer: 99911740.1
(22) Anmeldetag: 05.03.1999
(51) Int. Cl.: C07C 29/141, B01J 23/72

(54) **VERFAHREN ZUR HYDRIERUNG VON CARBONYLVERBINDUNGEN**
METHOD FOR HYDROGENATING CARBONYL COMPOUNDS
PROCEDE D'HYDROGENATION DE COMPOSES CARBONYLES

(30) Priorität: 05.03.1998 DE 19809418
(43) Veröffentlichungstag der Anmeldung: 20.12.2000
(73) Patentinhaber: BASF AKTIENGESELLSCHAFT, 67056 Ludwigshafen (DE)
(72) Erfinder: HESSE, Michael, D-67549 Worms (DE); KRATZ, Detlef, D-69121 Heidelberg (DE); SCHULZ, Gerhard, D-67069 Ludwigshafen (DE); WALTER, Marc, D-67227 Frankenthal (DE); SAUERWALD, Manfred, D-67149 Meckenheim (DE)
(74) Vertreter: Isenbruck, Günter
(86) Internationale Anmeldenummer: EP9901427
(87) Internationale Veröffentlichungsnummer: WO99044974

(56) Entgegenhaltungen:
- EP-A- 0 301 853
- WO-A-97/03937
- DE-A- 19 730 939
- US-A- 4 918 248

## Beschreibung

Die Erfindung betrifft ein Verfahren zur katalytischen Hydrierung von Carbonylverbindungen in Gegenwart eines Kupfer-Katalysators, den Kupferkatalysator an sich sowie ein Verfahren zu dessen Herstellung.

Die katalytische Hydrierung von Carbonylverbindungen wie z.B. Aldehyden zur Herstellung einfacher und funktionalisierter Alkohole nimmt in den Produktionssträngen der chemischen Grundstoffindustrie eine bedeutende Stellung ein. Besonders gilt dies für die Hydrierung von Aldehyden, die über Oxosynthese oder Aldolreaktion zugänglich sind.

Die katalytische Hydrierung von Aldehyden in Suspensions- oder Festbettfahrweise ist seit langem bekannt. Technische Anlagen arbeiten fast ausschließlich mit Festbettreaktoren.

Als Festbettkatalysatoren werden, neben Katalysatoren vom Raney-Typ (DE-A 197 30 939), jedoch vor allem geträgerte Katalysatoren, beispielsweise Kupfer-. Nickel- oder Edelmetall-Katalysatoren verwendet.

DE-A 16 43 856 beschreibt die Hydrierung von Aldehyden zu Alkoholen an kombinierten Cu/Ni-Trägerkatalysatoren in der Gasphase. Die verwendeten Träger müssen dabei vor Einsatz mit Alkalien neutralisiert werden.

DE-A 40 37 729 beschreibt ein als Katalysator zur Hydrierung von Fettsäuren bzw. Fettsäureestern eingesetztes Cu/Cr-System, das aufgrund der Chromkomponente im Hinblick auf die Umweltbelastung problematisch ist. Weiter erfordern diese Katalysatoren relativ drastische Reaktionsbedingungen beim Hydrieren, was zu einer vermehrten Bildung von unerwünschten Nebenprodukten führen kann.

Trägerkatalysatoren, die das bedenkliche Chrom nicht enthalten, beschreibt EP-A 0 044 444. Die in dem dort geschilderten Verfahren zur Herstellung von Propandiol eingesetzten. auf Al₂O₃ geträgerten Cu-Hydrierkatalysatoren zeichnen sich zwar durch hohe Anfangsaktivität aus, zeigen jedoch im Hinblick auf ihr Standzeitverhalten keine befriedigenden Eigenschaften.

In EP-A 0 484 800 wird die Verwendung von auf ZrO₂ geträgerten Cu/Zn-Katalysatoren zur Hydrierung von Hydroxypivalinaldehyd zu Neopentylglykol offenbart.

US 4 918 248 offenbart den Einsatz von auf TiO₂ geträgerten Cu/Zn-Katalysatoren, deren Verwendung ausschließlich auf die Hydrierung von Carbonsäureestern beschränkt ist.

Der Erfindung lag daher die Aufgabe zugrunde, ein Verfahren zur katalytischen Hydrierung von Carbonylverbindungen bereitzustellen, wobei ein Katalysator eingesetzt wird, der technisch in einfacher Weise herzustellen ist, der hohe mechanische Stabilität bei den im genannten Verfahren auftretenden Reaktionsbedingungen aufweist und hohe Umsätze und Selektivitäten ermöglicht.

Es wurde gefunden, daß eine Ausführungsform der Tablettierung, bei der einem getrockneten Pulver, das das Trägermaterial und die Aktivkomponente umfaßt, neben einem üblichen Tablettierungshilfsmittel wie z.B. Graphit zusätzlich metallisches Cu-Pulver beigemischt wird, sowohl zu einer besonders leicht durchzuführenden Tablettierung als auch zu hohen Aktivitäten und Selektivitäten sowie zu einer hohen Stabilität des Katalysators führt.
Eine herausragende Eigenschaft der so gewonnenen Katalysatortabletten ist die außerordentliche mechanische Festigkeit. Erst der erfindungsgemäß verwendete Zusatz von metallischem Cu-Pulver verleiht den Tabletten die mechanische Stabilität, die nötig ist, um mit dem gewählten Trägermaterial einen Katalysator herzustellen, der unter den Reaktionsbedingungen der katalytischen Hydrierung sinnvoll einsetzbar ist.

Demgemäß wurde die Aufgabe gelöst durch ein Verfahren zur katalytischen Hydrierung einer Carbonylverbindung oder eines Gemisches aus zwei oder mehr Carbonylverbindungen in Gegenwart eines Katalysators in Tablettenform, der einen anorganischen Träger, der TiO₂ enthält, und als Aktivkomponente Kupfer oder ein Gemisch aus Kupfer mit mindestens einem der Metalle, ausgewählt aus der Gruppe Zink, Aluminium, Cer, einem Edelmetall und einem Metall der VIII. Nebengruppe, umfaßt, dadurch gekennzeichnet, daß die spezifische Kupferoberfläche maximal 10 m²/g beträgt, und der Katalysator zusätzlich Kupfermetall in Pulverform enthält.

Bevorzugt wird als Träger TiO₂ oder eine Mischung aus TiO₂ und Al₂O₃ oder eine Mischung aus TiO₂ und ZrO₂ oder eine Mischung aus TiO₂, Al₂O₃ und ZrO₂, besonders bevorzugt TiO₂ verwendet.

Der in dem erfindungsgemäßen Verfahren verwendete Katalysator zeichnet sich dadurch aus, daß die Aktivkomponente Kupfer auf das verwendete Trägermaterial aufgebracht wird, wobei bzgl. der Aufbringungsmethode keinerlei Beschränkungen existieren.

Insbesondere kommen folgende Aufbringungsmethoden in Betracht:
a) Aufbringung eine Kupfersalzlösung in einer oder mehreren Tränkstufen auf einen vorgefertigten anorganischen Träger. Der Träger wird im Anschluß an die Tränkung getrocknet und ggf. calciniert.
   a1) Die Tränkung kann nach der sogenannten "incipient wetness"-Methode erfolgen, bei der der Träger entsprechend seiner Wasseraufnahmekapazität maximal bis zur Sättigung mit der Tränklösung befeuchtet wird. Die Tränkung kann aber auch in überstehender Lösung erfolgen.
   a2) Bei mehrstufigen Tränkverfahren ist es zweckmäßig, zwischen einzelnen Tränkschritten zu trocknen und ggf. zu calcinieren. Die mehrstufige Tränkung ist vorteilhaft besonders dann anzuwenden, wenn der Träger mit einer größeren Kupfermenge beaufschlagt werden soll.
   a3) Bevorzugt wird das anorganische Trägermaterial bei der Tränkung als vorgeformte Masse eingesetzt, beispielsweise als Pulver, Kugeln, Stränge oder Tabletten. Besonders bevorzugt wird der Einsatz als Pulver.
   a4) Als Lösungsmittel der Kupfersalze wird bevorzugt konzentrierter wäßriger Ammoniak eingesetzt.
b) Fällung einer Kupfersalzlösung auf einen vorgefertigten, inerten anorganischen Träger. Dieser liegt in einer besonders bevorzugten Ausführungsform als Pulver in einer wäßrigen Suspension vor.
   b1) In einer Ausführungsform (i) wird eine Kupfersalzlösung, bevorzugt mit Sodalösung, gefällt. Als Vorlage wird eine wäßrige Suspension des Trägermaterials verwendet.
   b2) In einer weiteren Ausführungsform (ii) kann der Fällkatalysator in einem Zwei-Stufen-Prozeß hergestellt werden. Dabei wird in einer ersten Stufe ein Pulver gemäß den Angaben aus a) hergestellt und getrocknet. Dieses Pulver wird in eine wäßrige Suspension überführt und als Vorlage äquivalent zu der in Ausführungsform (i) beschriebenen eingesetzt.
   Ausgefällte Niederschläge, die aus a) oder b) resultieren, werden in üblicher Weise filtriert und vorzugsweise alkalifrei gewaschen.

Sowohl die Endprodukte aus a) als auch die aus b) werden bei Temperaturen von 50 bis 150 °C, vorzugsweise bei 120 °C getrocknet und im Anschluß ggf. vorzugsweise 2 Stunden bei im allgemeinen 200 bis 400 °C, insbesondere bei 200 bis 220 °C calciniert.

Als Ausgangssubstanzen für a) und/oder b) können prinzipiell alle in den bei der Aufbringung verwendeten Lösungsmitteln löslichen Cu(I) und/oder Cu(II)-Salze, beispielsweise Sulfate, Nitrate, Chloride, Carbonate, Acetate, Oxalate oder Ammonium-Komplexe, verwendet werden. Besonders bevorzugt für Verfahren gemäß a) wird Kupfercarbonat eingesetzt, für Verfahren gemäß b) Kupfernitrat.

In dem erfindungsgemäßen Verfahren wird das oben beschriebene getrocknete Pulver bevorzugt zu Tabletten oder ähnlichen Formkörpern verformt. Als Tablettierungshilfsmittel wird für den Verformungsprozeß Graphit, vorzugsweise in einem Anteil von 3 Gew.-%, bezogen auf das Gewicht des getrockneten Pulvers, zugegeben.

Als weiteres Additiv wird zur Herstellung des Katalysators, zusätzlich zum oben beschriebenen Pulver und zu Graphit, metallisches Cu-Pulver zugesetzt. Bevorzugt werden, bezogen auf das Gewicht des oben beschriebenen getrockneten Pulvers, 5 bis 40 Gew.-% metallisches Cu-Pulver zugesetzt, insbesondere 15 bis 20 Gew.-%.

Demnach betrifft die vorliegende Erfindung auch einen Katalysator, der einen anorganischen Träger, der TiO₂ enthält, und als Aktivkomponente Kupfer oder ein Gemisch aus Kupfer mit mindestens einem der Metalle, ausgewählt aus der Gruppe Zink, Aluminium, Cer, einem Edelmetall und einem Metall der VIII. Nebengruppe, umfaßt, erhältlich durch ein Verfahren, das einen Tablettierungsschritt umfaßt, dadurch gekennzeichnet, daß bei der Tablettierung metallisches Kupferpulver zugesetzt wird.

Die erfindungsgemäßen Tablettenformkörper werden, vorzugsweise 2 Stunden, bei 300 bis 600 °C, insbesondere bei 330 bis 350 °C getempert. Dieses neuartige Verfahren zur Tablettierung erlaubt, im Vergleich zum ausschließlichen Einsatz von Graphit als Tablettierungshilfsmittel in den üblichen Verfahren. eine besonders leicht durchzuführende Verformung des Pulvers zu Tabletten und liefert sehr chemisch und mechanisch stabile Katalysatoren.

Die Parameter "Härte" und "Abrieb" können wie folgt bestimmt werden. Zur Ermittlung der Schneidhärte werden Proben mit einer Schneide zertrennt. Die Kraft, mit der die Schneide belastet werden muß. um eine Durchtrennung der Probe zu erreichen, bezeichnet man als Schneidhärte des Materials.

Die Bruchhärte von kugelförmigen Proben wird dadurch ermittelt, daß man die Kugel unter einen Stempel mit definierter Fläche legt und den Stempel dann solange gegen die Kugel bewegt, bis sie zerbricht. Der mit dem Stempel auf die Probe ausgeübte Druck, der zur Erzielung des Bruches notwendig ist, wird als Bruchhärte bezeichnet.

Der Abrieb wird mit einer Schwingmühle bestimmt. Dabei wird Katalysatormaterial eines bestimmten Komgrößenbereichs zusammen mit Porzellankugeln in einem Behälter bei hoher Drehzahl für eine bestimmte Zeitdauer bewegt. Danach wird der Katalysator wieder abgesiebt. Der Gewichtsverlust in % wird dann als Abrieb bezeichnet, wie dies in Kapitel 6 in J.-F. Le Page et al., "Applied Heterogeneous Catalysis", Editions Technip, Paris (1987), beschrieben ist.

Das Aktivieren des geglühten Katalysators erfolgt entweder vor oder nach dem Einbau in den Reaktor.

Soll der Katalysator in seiner reduzierten Form verwendet werden, wird er in den Reaktor eingebaut und direkt unter Wasserstoffdruck mit der Hydrierlösung beschickt. Bei Einsatz in der oxidischen Form wird der Katalysator vor Beschickung mit der Hydrierlösung mit reduzierenden Gasen. beispielsweise Wasserstoff, vorzugsweise Wasserstoff-Inertgasgemischen. insbesondere Wasserstoff/Stickstoffgemischen bei Temperaturen von 100 bis 300, bevorzugt von 150 bis 250, insbesondere von 180 bis 240 °C vorreduziert. Bevorzugt wird dabei ein Gemisch mit einem Wasserstoffanteil von 1 bis 100 Vol.-% verwendet.

Eine charakteristische Größe der erfindungsgemäßen Katalysatoren ist deren spezifische Kupferoberfläche. Sie wird aus dem durch Oxidation von Oberflächen-Kupfer-Atomen mit gasförmigem N₂O in einer erhitzten Probe des Katalysators ermittelten N₂O-Verbrauch berechnet.

Dazu wird die Probe zunächst 4 Stunden mit 10 mbar Wasserstoff bei einer Temperatur von 240 °C behandelt. Anschließend wird die Probe bis zu einem Druck von kleiner als 10⁻³ mbar evakuiert und danach 3 Stunden mit 30 mbar H₂ behandelt, anschließend nochmals auf kleiner 10⁻³ mbar evakuiert, 3 Stunden mit 100 mbar H₂ behandelt, wiederum auf kleiner 10⁻³ mbar evakuiert und abschließend nochmals 3 Stunden mit 200 mbar H₂ behandelt, wobei die Behandlung mit Wasserstoff jeweils bei einer Temperatur von 240 °C durchgeführt wurde.

In einer zweiten Stufe wird die Probe mit N₂O bei einer Temperatur von 70 °C bei einem Druck von 266 mbar 2 Stunden lang beaufschlagt, wobei eine Zersetzung des N₂O an der Probe zu beobachten ist. Anschließend wird die Probe auf kleiner 10⁻³ mbar evakuiert und danach die Zunahme der Masse des Katalysators infolge der Bildung von Kupferoxid an der Oberfläche desselben bestimmt.

Die so gemessene spezifische Kupfer-Oberfläche der erfindungsgemäß hergestellten Katalysatoren beträgt im allgemeinen maximal 10 m²/g, vorzugsweise 0,1 bis 10 m²/g, weiter bevorzugt liegt sie im Bereich von 0,5 bis 7 m²/g, insbesondere im Bereich von 0,5 bis 5 m²/g.
Demgemäß betrifft die vorliegende Erfindung auch einen Katalysator, der einen anorganischen Träger, der TiO₂ enthält, und als Aktivkomponente Kupfer oder ein Gemisch aus Kupfer mit mindestens einem der Metalle, ausgewählt aus der Gruppe Zink, Aluminium, Cer, einem Edelmetall und einem Metall der VIII. Nebengruppe, umfaßt, dadurch gekennzeichnet, daß die Kupferoberfläche maximal 10 m²/g beträgt.

Darüberhinaus betrifft die vorliegende Erfindung demnach auch ein Verfahren zur Herstellung eines Katalysators, der einen anorganischen Träger, der TiO₂ enthält, und als Aktivkomponente Kupfer oder ein Gemisch aus Kupfer mit mindestens einem der Metalle, ausgewählt aus der Gruppe Zink, Aluminium, Cer. einem Edelmetall und einem Metall der VIII. Nebengruppe, umfaßt, und dessen Kupferoberfläche maximal 10 m²/g beträgt, das eine Tablettierung umfaßt, dadurch gekennzeichnet, daß bei der Tablettierung metallisches Kupferpulver zugesetzt wird.

Bevorzugtes Einsatzgebiet der erfindungsgemäß hergestellten Katalysatoren ist die Hydrierung im Festbett. Die Ausführungsform als Wirbelbettreaktion mit in auf- und abwirbelnder Bewegung befindlichem Katalysatormaterial ist jedoch ebenfalls möglich. Die Hydrierung kann in der Gasphase oder in der Flüssigphase durchgeführt werden. Vorzugsweise wird die Hydrierung in flüssiger Phase durchgeführt, beispielsweise in Riesel- oder Sumpffahrweise.

Bei Arbeiten in Rieselfahrweise läßt man das flüssige, die zu hydrierende Carbonylverbindung enthaltende Edukt in dem Reaktor, der unter Wasserstoffdruck steht, über das in diesem angeordnete Katalysatorbett rieseln, wobei sich auf dem Katalysator ein dünner Flüssigkeitsfilm ausbildet. Dagegen wird beim Arbeiten in Sumpffahrweise Wasserstoffgas in den mit der flüssigen Reaktionsmischung gefluteten Reaktor eingeleitet, wobei der Wasserstoff das Katalysatorbett in aufsteigenden Gasperlen passiert.

In einer Ausführungsform wird die zu hydrierende Lösung im geraden Durchgang über die Katalysatorschüttung gepumpt. In einer anderen Ausführungsform des erfindungsgemäßen Verfahrens wird ein Teil des Produkts nach Durchgang durch den Reaktor als Produktstrom kontinuierlich abgezogen und ggf. durch einen zweiten Reaktor, wie oben definiert, geleitet. Der andere Teil des Produkts wird zusammen mit frischem, die Carbonylverbindung enthaltendem Edukt dem Reaktor erneut zugeführt. Diese Verfahrensweise wird im folgenden als Kreislauffahrweise bezeichnet.

Wird als Ausführungsform des erfindungsgemäßen Verfahrens die Rieselfahrweise gewählt, ist hierbei die Kreislauffahrweise bevorzugt. Weiter bevorzugt wird in Kreislauffahrweise unter Verwendung eines Haupt- und Nachreaktors gearbeitet.

Das erfindungsgemäße Verfahren eignet sich zur Hydrierung von Carbonylverbindungen wie z.B. Aldehyden und Ketonen zu den entsprechenden Alkoholen, wobei aliphatische und cycloaliphatische gesättigte und ungesättigte Carbonylverbindungen bervorzugt sind. Bei aromatischen Carbonylverbindungen kann es zur Bildung unerwünschter Nebenprodukte durch Hydrierung des aromatischen Kerns kommen. Die Carbonylverbindungen können weitere funktionelle Gruppen wie Hydroxy- oder Aminogruppen tragen. Ungesättigte Carbonylverbindungen werden in der Regel zu den entsprechenden gesättigten Alkohlen hydriert. Der Begriff "Carbonylverbindungen", wie er im Rahmen der Erfindung verwendet wird, umfaßt alle Verbindungen, die eine C=O-Gruppe aufweisen, einschließlich Carbonsäuren und deren Derivaten. Selbstverständlich können auch Gemische aus zwei oder mehr als zwei Carbonylverbindungen gemeinsam hydriert werden. Ferner kann auch die einzelne, zu hydrierende Carbonylverbindung mehr als eine Carbonylgruppe enthalten.

Bevorzugt wird das erfindungsgemäße Verfahren zur Hydrierung aliphatischer Aldehyde, Hydroxyaldehyde, Ketone, Säuren. Ester, Anhydride, Lactone und Zucker eingesetzt.

Bevorzugte aliphatische Aldehyde sind verzweigte und unverzweigte gesättigte und/oder ungesättigte aliphatische C₂-C₃₀-Aldehyde, wie sie beispielsweise durch Oxosynthese aus linearen oder verzweigten Olefinen mit interner oder terminaler Doppelbindung erhältlich sind. Femer können auch oligomere Verbindungen, die auch mehr als 30 Carbonylgruppen enthalten, hydriert werden.

Als Beispiel für aliphatische Aldehyde sind zu nennen:
Formaldehyd, Propionaldehyd, n-Butyraldehyd, iso-Butyraldehyd, Valeraldehyd, 2-Methylbutyraldehyd, 3-Methylbutyraldehyd (Isovaleraldehyd), 2,2-Dimethylpropionaldehyd (Pivalinaldehyd), Capronaldehyd, 2-Methylvaleraldehyd, 3-Methylvaleraldehyd, 4-Methylvaleraldehyd, 2-Ethylbutyraldehyd, 2,2-Dimethylbutyraldehyd, 3,3-Dimethylbutyraldehyd, Caprylaldehyd, Caprinaldehyd, Glutardialdehyd.

Neben den genannten kurzkettigen Aldehyden sind insbesondere auch langkettige aliphatische Aldehyde geeignet, wie sie beispielsweise durch Oxosynthese aus linearen α-Olefinen erhalten werden können.

Besonders bevorzugt sind Enalisierungsprodukte, wie z.B. 2-Ethylhexenal, 2-Methylpentenal, 2,4-Diethyloctenal oder 2,4-Dimethylheptenal.

Bevorzugte Hydroxyaldehyde sind C₃-C₁₂-Hydroxyaldehyde, wie sie beispielsweise durch Aldolreaktion aus aliphatischen und cycloaliphatischen Aldehyden und Ketonen mit sich selbst oder Formaldehyd zugänglich sind. Beispiele sind 3-Hydroxypropanal, Dimethylolethanal, Trimethylolethanal (Pentaerythrital), 3-Hydroxybutanal (Acetaldol), 3-Hydroxy-2-ethylhexanal (Butylaldol), 3-Hydroxy-2-methylpentanal (Propienaldol), 2-Methylolpropanal, 2,2-Dimethylolpropanal, 3-Hydroxy-2-methylbutanal, 3-Hydroxypentanal, 2-Methylolbutanal, 2,2-Dimethylolbutanal, Hydroxypivalinaldehyd. Besonders bevorzugt sind Hydroxypivalinaldehyd (HPA) und Dimethylolbutanal (DMB).

Bevorzugte Ketone sind Aceton, Butanon, 2-Pentanon, 3-Pentanon, 2-Hexanon, 3-Hexanon, Cyclohexanon, Isophoron, Methylisobutylketon, Mesityloxid, Acetophenon, Propiophenon, Benzophenon, Benzalaceton, Dibenzalaceton, Benzalacetophenon, 2,3-Butandion, 2,4-Pentandion, 2,5-Hexandion und Methylvinylketon.

Darüber hinaus können Carbonsäuren und Derivate davon, vorzugsweise solche mit 1-20 C-Atomen umgesetzt werden. Insbesonderes sind die folgenden zu nennen:
Carbonsäuren, wie z.B. Ameisensäure, Essigsäure, Propionsäure, Buttersäure, Isobuttersäure, n-Valeriansäure, Trimethylessigsäure ("Pivalinsäure"), Capronsäure, Önanthsäure, Caprylsäure, Caprinsäure, Laurinsäure, Myristinsäure, Palmitinsäure, Stearinsäure, Acrylsäure, Methacrylsäure, Ölsäure, Elaidinsäure, Linolsäure, Linolensäure, Cyclohexancarbonsäure, Benzoesäure, Phenylessigsäure, o-Toluylsäure, m-Toluylsäure, p-Toluylsäure. o-Chlorbenzoesäure, p-Chlorbenzoesäure, o-Nitrobenzoesäure, p-Nitrobenzoesäure, Salicylsäure, p-Hydroxybenzoesäure, Anthranilsäure. p-Aminobenzoesäure, Oxalsäure, Malonsäure, Bernsteinsäure, Glutarsäure, Adipinsäure, Pimelinsäure, Korksäure, Azelainsäure, Sebacinsäure, Maleinsäure, Fumarsäure, Phthalsäure, Isophthalsäure, Terephthalsäure;
Carbonsäurehalogenide, wie z.B. die Chloride oder Bromide der oben genannten Carbonsäuren, insbesondere Acetylchlorid oder -bromid, Stearylchlorid oder -bromid und Benzoylchlorid oder -bromid. die insbesondere dehalogeniert werden;
Carbonsäureester, wie z.B. die C₁-C₁₀-Alkylester der oben genannten Carbonsäuren, insbesondere Methylformiat, Essigester, Buttersäurebutylester, Terephthalsäuredimethylester. Adipinsäuredimethylester, Maleinsäuredimethylester, (Meth)acrylsäuremethylester, Butyrolacton, Caprolacton und Polycarbonsäureester, wie z.B. Polyacryl- und Polymethacrylsäureester und deren Copolymere und Polyester, wie z.B. Polymethylmethacrylat, Terephthalsäureester und andere technische Kunststoffe, wobei hier insbesondere Hydrogenolysen, also die Umsetzung von Estern zu den entsprechenden Säuren und Alkoholen, durchgeführt werden;
Fette;
Carbonsäureanhydride, wie z.B. die Anhydride der oben genannten Carbonsäuren. insbesondere Essigsäureanhydrid. Propionsäureanhydrid, Benzoesäureanhydrid und Maleinsäureanhydrid;
Carbonsäureamide, wie z.B. Formamid, Acetamid, Propionamid, Stearamid, Terephthalsäureamid.

Ferner können auch Hydroxycarbonsäuren, wie z.B. Milch-, Äpfel-, Wein- oder Zitronensäure, oder Aminosäuren, wie z.B. Glycin, Alanin, Prolin und Arginin, und Peptide umgesetzt werden.

Besonders bevorzugt wird das erfindungsgemäße Verfahren zur Hydrierung von Aldehyden und Hydroxyaldehyden eingesetzt.

Die zu hydrierende Carbonylverbindung kann dem Hydrierungsreaktor allein oder als Gemisch mit dem Produkt der Hydrierungsreaktion zugeführt werden, wobei dies in unverdünnter Form oder unter Verwendung von zusätzlichem Lösungsmittel geschehen kann. Als zusätzliches Lösungsmittel eigenen sich insbesondere Wasser, Alkohole wie Methanol, Ethanol und der Alkohol, der unter den Reaktionsbedingungen entsteht. Bevorzugte Lösungsmittel sind Wasser, THF, NMP, sowie Ether, wie z.B. Dimethyl-, Diethylether, MTBE, besonders bevorzugt ist Wasser.

Die Hydrierung sowohl in Sumpf- als auch in Rieselfahrweise, wobei jeweils bevorzugt in Kreislauffahrweise gearbeitet wird, führt man im allgemeinen bei einer Temperatur von 50 bis 250 °C, bevorzugt bei 70 bis 200 °C, besonders bevorzugt bei 100 bis 140 °C und einem Druck von 15 bis 250 bar, bevorzugt 20 bis 200 bar, besonders bevorzugt 25 bis 100 bar durch.

Mit dem erfindungsgemäßen Verfahren werden hohe Umsätze und Selektivitäten erzielt, und die Katalysatoren weisen eine hohe chemische Stabilität in Gegenwart des Reaktionsgemisches auf. Bei gleichem Trägermaterial zeigen die erfindungsgemäß hergestellten Katalysatoren gegenüber denjenigen Katalysatoren, die nach dem Stand der Technik hergestellt werden, sowohl eine leichtere Verformbarkeit zu Tabletten als auch, nach dem Tempern der Tablettenformkörper, eine sowohl im oxidischen als auch im reduzierten Zustand deutlich höhere mechanische Stabilität, wodurch sich das erfindungsgemäße Verfahren durch eine besondere Wirtschaftlichkeit auszeichnet.

Die Erfindung wird in den nachstehenden Beispielen näher erläutert.

### Beispiele

### Katalysatorherstellung

Alle unter diesem Unterpunkt angegebenen Prozentangaben stellen, soweit nicht anders vermerkt, Gewichtsprozente dar. Die bei den einzelnen Katalysatoren angegebenen Kupferoxidkristallitgrößen wurden mittles XRD ermittelt. Die angegebenen prozentualen Zusammensetzungen beziehen sich auf die oxidischen Bestandteile der fertigen Katalysatoren.

### Katalysator A (Vergleich)

Katalysator A wurde durch Fällung einer Lösung von Kupfer und Aluminiumnitrat mit Sodalösung hergestellt. Das entstehende Fällgut wurde abfiltriert, gewaschen und bei 120 °C getrocknet. Das getrocknete Pulver wurde zwei Stunden bei 250 °C calciniert und danach mit 3 % Graphit zu Tabletten mit 5 mm Durchmesser verpreßt. Diese Tabletten wurden 2 Stunden bei 580 °C getempert. Der fertige Katalysator enthielt 53 % CuO und 47 % Al₂O₃, bei einem Rüttelgewicht von 1090 g/l, einer Oberfläche gemäß BET von 101 m²/g bzw. 110090 m²/l, einer Kupferoberfläche von 11.5 m²/g bzw. 11445 m²/l, einer Kupferoxidkristallitgröße von 15,0 nm und einer Wasseraufnahme von 0,41 ml/g bzw. 447 ml/l.

### Katalysator B (Vergleich)

Katalysator B wurde hergestellt durch Tränkung von SiO₂-Kugeln von 3 bis 5 mm Durchmesser mit einer Lösung von Kupfercarbonat in konzentriertem wäßrigem Ammoniak. Die Tränkung erfolgte 15 min in überstehender Lösung. Die getränkten Kugeln wurden 5 Stunden bei 120 °C getrocknet und danach 2 Stunden bei 250 °C calciniert. Diese Tränk- und Calcinierschritte wurden wiederholt. Der fertige Katalysator enthielt 25,6 % CuO und 74,4 % SiO₂, bei einem Rüttelgewicht von 605 g/l, einer Oberfläche gemäß BET von 212 m²/g bzw. 128260 m²/l, einer Kupferoberfläche von 9,8 m²/g bzw. 5929 m²/l, einer Kupferoxidkristallitgröße von 2,5 nm und einer Wasseraufnahme von 0,54 ml/g bzw. 327 ml/l.

### Katalysator C (Vergleich)

Katalysator C wurde hergestellt durch Tränkung von TiO₂-Pulver mit einer Lösung von Kupfercarbonat in konzentriertem wäßrigem Ammoniak. Die Tränkung erfolgte zuerst bei Raumtemperatur, dann bei 100 °C. Das so getränkte Pulver wurde bei 120 °C getrocknet und danach unter Zugabe von 3% Graphit tablettiert. Die Tabletten von 3 mm Durchmesser und 3 mm Höhe wurden zwei Stunden bei 350 °C getempert. Der fertige Katalysator enthielt 25 % CuO und 75 % TiO₂, bei einem Rüttelgewicht von 1216 g/l, einer Oberfläche gemäß BET von 91 m²/g bzw. 110656 m²/l, einer Kupferoberfläche von 0,3 m²/g bzw. 365 m²/l, einer Kupferoxidkristallitgröße von 13,5 nm und einer Wasseraufnahme von 0,29 ml/g bzw. 353 ml/l.

### Katalysator D

Katalysator D wurde hergestellt wie Katalysator C. jedoch wurden bei der Tablettierung zusätzlich 15 % metallisches Kupferpulver zugesetzt. Der fertige Katalysator enthielt 60 % TiO₂, bei einem Rüttelgewicht von 1508 g/l, einer Oberfläche gemäß BET von 65 m²/g bzw. 98020 m²/l, einer Kupferoberfläche von 0,7 m²/g bzw. 1055 m²/l, einer Kupferoxidkristallitgröße von 17,5 nm und einer Wasseraufnahme von 0,22 ml/g bzw. 323 ml/l.

### Katalysator E (Vergleich)

Katalysator E wurde durch Tablettierung von TiO₂-Pulver mit 3 % Graphit und 40 % Kupferpulver hergestellt. Die Tabletten von 3 mm Durchmesser und 3 mm Höhe wurden zwei Stunden bei 350 °C getempert. Der fertige Katalysator enthielt 60 % TiO₂, bei einem Rüttelgewicht von 1940 g/l, einer Oberfläche gemäß BET von 32 m²/g bzw. 62080 m²/l, einer Kupferoberfläche von 0,5 m²/g bzw. 970 m²/l, einer Kupferoxidkristallitgröße von 14,0 nm und einer Wasseraufnahme von 0,08 ml/g bzw. 155 ml/l.

### Katalysator F (Vergleich)

Katalysator F wurde durch Fällung einer Lösung von Kupfernitrat mit Sodalösung hergestellt. Als Vorlage wurde eine Suspension von TiO₂ in Wasser verwendet. Das bei der Fällung entstehende Fällgut wurde abfiltriert, gewaschen und bei 120 °C getrocknet. Das getrocknete Pulver wurde zwei Stunden bei 200 °C calciniert und danach mit 3 % Graphit zu Tabletten mit 3 mm Durchmesser verpreßt. Diese Tabletten wurden 2 Stunden bei 330 °C getempert. Der fertige Katalysator enthielt 53 % CuO und 47 % TiO₂, bei einem Rüttelgewicht von 1900 g/l, einer Oberfläche gemäß BET von 74 m²/g bzw. 140600 m²/l, einer Kupferoberfläche von 2,2 m²/g bzw. 4180 m²/l, einer Kupferoxidkristallitgröße von 15,5 nm und einer Wasseraufnahme von 0,28 ml/g bzw. 532 ml/l.

### Katalysator G (Vergleich)

Katalysator G wurde durch Fällung einer Lösung von Kupfernitrat mit Sodalösung hergestellt. Als Vorlage wurde eine Suspension von TiO₂ und Al₂O₃ in Wasser verwendet. Das bei der Fällung entstehende Fällgut wurde abfiltriert, gewaschen und bei 120 °C getrocknet. Das getrocknete Pulver wurde zwei Stunden bei 220 °C calciniert und danach mit 3 % Graphit zu Tabletten mit 3 mm Durchmesser verpreßt. Diese Tabletten wurden zwei Stunden bei 330 °C getempert. Der fertige Katalysator enthielt 56 % CuO, 12 % Al₂O₃ und 32 % TiO₂, bei einem Rüttelgewicht von 1420 g/l, einer Oberfläche gemäß BET von 77 m²/g bzw. 109340 m²/l, einer Kupferoberfläche von 3,6 m²/g bzw. 5112 m²/l, einer Kupferoxidkristallitgröße von 19,5 nm und einer Wasseraufnahme von 0,24 ml/g bzw. 341 ml/l.

### Katalysator H (Vergleich)

Katalysator H wurde durch Fällung einer Lösung von Kupfernitrat mit Sodalösung hergestellt. Als Vorlage wurde eine Suspension von TiO₂ in Wasser verwendet. Das bei der Fällung enstehende Fällgut wurde abfiltriert, gewaschen und bei 120 °C getrocknet. Das getrocknete Pulver wurde zwei Stunden bei 220 °C calciniert und danach mit 3 % Graphit zu Tabletten mit 3 mm Durchmesser verpreßt. Diese Tabletten wurden 2 Stunden bei 330 °C getempert. Der fertige Katalysator enthielt 30 % CuO und 70 % TiO₂, bei einem Rüttelgewicht von 1760 g/l, einer Kupferoberfläche von 1,3 m²/g bzw. 2288 m²/l, einer Kupferoxidkristallitgröße von 15,5 nm und einer Wasseraufnahme von 0,20 ml/g bzw. 352 ml/l.

### Katalysator I (Vergleich)

Katalysator I wurde hergestellt wie Katalysator H, nur daß statt einer wäßrigen TiO₂-Suspension eine Suspension von Al₂O₃ und Wasser als Vorlage bei der Fällung verwendet wurde. Der fertige Katalysator enthielt 53 % CuO und 47 % Al₂O₃, bei einem Rüttelgewicht von 1200 g/l und einer Wasseraufnahme 0.35 ml/g bzw. 420 ml/l.

### Katalysator J

Der erfindungsgemäße Katalysator J wurde hergestellt wie Katalysator F, nur daß bei Tablettierung zusätzlich 20 % metallisches Kupferpulver zugesetzt wurden. Der fertige Katalysator enthielt ca. 39 % TiO₂, bei einer Oberfläche gemäß BET von 13 m²/g bzw. 23660 m²/l, einer Kupferoberfläche von 1,2 m²/g bzw. 2184 m²/l, einer Kupferoxidkristallitgröße von 18,5 nm und einer Wasseraufnahme von 0,13 ml/g bzw. 237 ml/l.

### Beispiel 1

### Hydrierung von Hydroxypivalinaldehyd (HPA) zu Neopentylglykol (NPG) in Rieselfahrweise (Kreislauffahrweise)

Als Ausgangslösung diente ein Gemisch aus 38 % HPA, 38 % NPG und 24 % Wasser. Dieses Gemisch wurde in einem Reaktor des Volumens 200 ml in Kreislauffahrweise bei einem Durchsatz von 9,5 l/h, einem Druck von 35 bar und einer Temperatur von 130 °C jeweils mit den Katalysatoren A bis I hydriert. Die Katalysatorbelastung betrug jeweils 0.35 l_{HPA}/(l_{Kat}h).

Der Vergleich des erfindungsgemäßen Katalysators D mit den Katalysatoren A bis C und E bis G (siehe Tabelle 1) zeigt, daß die bei Einsatz von D erzielten Umsätze hoch sind. Gleiches gilt für die Selektivitäten.beim Vergleich von D mit A, B. F und G. Die Verunreinigungen im Austrag bei den Katalysatoren B bis G weisen weiter auf die hohe chemische Stabilität des Katalysators D hin.

Der Vergleich des erfindungsgemäßen Katalysators D mit den Katalysatoren A und E bis I zeigt die hohe mechanische Härte von D im reduziert-feuchten Zustand, der Vergleich von D mit allen übrigen Vergleichskatalysatoren die hohe mechanische Härte von D im oxidierten Zustand.

Der Vergleich der Katalysatoren D und C, die bis auf die Beimengung von metallischem Kupferpulver bei der Tablettierung von D identisch hergestellt wurden, hinsichtlich Umsatz und mechanischer Härte im oxidierten Zustand zeigt weiter die Vorteile des erfindungsgemäß hergestellten Katalysators.

### Beispiel 2

### Hydrierung von Dimethylolbutanal (DMB) zu Trimethylolpropan in Rieselfahrweise mit Kreislauf und Nachreaktor

Als Ausgangslösung diente ein Gemisch aus 65 % DMB und 35 % Wasser. Dieses Gemisch wurde in einem Reaktor des Volumens 210 ml (130 ml Hauptreaktor und 80 ml Nachreaktor) in Kreislauffahrweise bei einem Durchlauf von 7.5 l/h und einer Temperatur von 120 °C (Hauptreaktor) bzw. 130 °C (Nachreaktor) bei 90 bar mittels der Katalysatoren A, B, D und F hydriert, wobei die Katalysatorbelastung 0,3 kg_{DMB}/(l_{Kat}h) betrug.

Der Vergleich des erfindungsgemäßen Katalysators D mit den Katalysatoren A, B und F gemäß Tabelle 2 zeigt wieder die hohen Umsätze und Selektivitäten von D. Dies gilt für den Vergleich mit nach dem Stand der Technik hergestellten Katalysatoren mit unterschiedlichem Trägermaterial (Katalysatoren A und B) ebenso wie für den Vergleich mit TiO₂-geträgerten Katalysatoren, die nach dem Stand der Technik tablettiert wurden (Katalysator F).

### Beispiel 3

### Hydrierung von Hydroxypivalinaldehyd (HPA) zu Neopentylglykol (NPG) in Rieselfahrweise mit Kreislauf und Nachreaktor

Beispiel 3 wurde durchgeführt wie Beispiel 2. Anstelle des Druckes von 90 bar wurde jedoch bei einem Druck von 35 bar und anstelle einer Katalysatorbelastung von 0,3 kg_{DMB}/(l_{Kat}h) bei einer Katalysatorbelastung von 0,45 kg_{HPA}/(l_{Kat}h) gearbeitet. Hydriert wurde mittels der Katalysatoren A, B und J.

Ein Vergleich des erfindungsgemäßen Katalysators J mit A und B gemäß Tabelle 3 zeigt, daß J hohen Umsatz, hohe Selektivität und hohe Ausbeute gewährleistet.

Insbesondere ist die hohe chemische Stabilität von J unter den gewählten Versuchsbedingungen bemerkenswert, die beim nach dem Stand der Technik hergestellten Katalysator B beispielsweise zur Auflösung des Trägermaterials führen.

Im Vergleich zum Katalysator A zeichnet sich J hinsichtlich der Härte sowohl im reduziert-feuchten als auch im oxidierten Zustand und hinsichtlich des Abriebs durch die hohe mechanische Stabilität aus, wodurch Schlammbildung infolge Katalysatorabriebs, wie sie im Falle von A nach längerer Laufzeit auftritt, vermieden werden kann.

Sämtliche aufgeführten Vergleichskriterien tragen entscheidend zu einer größeren Wirtschaftlichkeit des Verfahrens beim Einsatz von J bei.

**Tabelle 1:**

| zu Beispiel 1 (Rieselfahrweise / Kreislauffahrweise) | | | | | | | | | |
|---|---|---|---|---|---|---|---|---|---|
| **Katalysator** | **A** | **B** | **C** | **D** | **E** | **F** | **G** | **H** | **I** |
| **Beimengung Kupferpulver bei Tablettierung/Gew.-%** | --- | --- | --- | 15 | --- | --- | --- | --- | --- |
| **Umsatz/% (aus GC-Flächen-%)** | 86,8 | 94,0 | 64,6 | 92,8 | 49,9 | 96,4 | 95,7 | n.e. | n.e. |
| **Selektivität / % (aus GC-Flächen-%)** | 89,1 | 92,5 | n.e. | 92,3 | n.e. | 92,8 | 93,7 | n.e. | n.e. |
| **Verunreinigung im Austrag / ppm** | n.e. | Cu: < 3 Si: 6 | Cu: < 3 Ti: < 3 | Cu: < 3 Ti: < 3 | Cu: < 3 Ti: < 3 | Cu: < 3 Ti: < 3 | Cu: < 3 Al: < 3 Ti: < 3 | n.e. | n.e. |
| **Härte/N (reduziert-feuchter Zustand)** | < 15 | n.e. | n.e. | 21 | < 15 | <15 | < 15 | < 15 | < 15 |
| **Härte / N (oxidierter Zustand)** | 57 | 47 | 61 | 130 | 92 | 58 | 80 | 61 | 75 |
| **Abrieb / Gew.-% (oxidierter Zustand)** | 5,1 | 6,4 | 3,5 | 5,2 | 2,9 | 7,3 | 13 | 1,5 | 9,0 |

**Tabelle 2:**

| zu Beispiel 2 (Rieselfahrweise mit Kreislauf und Nachreaktor) | | | | |
|---|---|---|---|---|
| **Katalysator** | **A** | **B** | **D** | **F** |
| **Beimengung Kupferpulver bei Tablettierung/ Gew.- %** | --- | --- | 15 | --- |
| **Umsatz/% (aus GC-Flächen-%)** | 96,2 | 99,3 | 100 | 100 |
| **Selektivität / % (aus GC-Flächen-%)** | 73,4 | 78,4 | 83,6 | 85,6 |

**Tabelle 3:**

| zu Beispiel 3 (Rieselfahrweise mit Kreislauf und Nachreaktor) | | | |
|---|---|---|---|
| **Katalysator** | **A** | **B** | **J** |
| **Beimengung Kupferpulver bei Tablettierung/ Gew.- %** | **---** | **---** | 20 |
| **Umsatz/% (aus GC-Flächen-%)** | 76,8 | 98,6 | 99,4 |
| **Selektivität /% (aus GC-Flächen-%)** | 85,1 | 96,0 | 96,5 |
| **Ausbeute /%** | 65,4 | 94,6 | 95,9 |
| **Verunreinigung im Austrag/ppm** | Cu: n.e. Al: n.e. | Cu: < 1 Si: 6 (Auflösung d. Trägers) | Cu: < 3 Ti: < 3 |
| **Härte / N (reduziert-feuchter Zustand)** | < 15 | n.e. | 58 |
| **Härte / N (oxidierter Zustand)** | 57 | 47 | 92 |
| **Abrieb / Gew.-% (oxidierter Zustand)** | 5,1 | 6,4 | 0,7 |

## Patentansprüche

1. Verfahren zur katalytischen Hydrierung einer Carbonylverbindung oder eines Gemisches aus zwei oder mehr Carbonylverbindungen in Gegenwart eines Katalysators in Tablettenform, der einen anorganischen Träger, der TiO₂ enthält, und als Aktivkomponente Kupfer oder ein Gemisch aus Kupfer mit mindestens einem der Metalle, ausgewählt aus der Gruppe Zink, Aluminium, Cer, einem Edelmetall und einem Metall der VIII. Nebengruppe, umfaßt, worin der Katalysator zusätzlich Kupfermetall in Pulverform enthält und die spezifische Kupferoberfläche maximal 10 m²/g beträgt.

2. Verfahren nach Anspruch 1, **dadurch gekennzeichnet, daß** das Trägermaterial eine Mischung aus TiO₂ und Al₂O₃ oder eine Mischung aus TiO₂ und ZrO₂ oder eine Mischung aus TiO₂ und Al₂O₃ und ZrO₂ umfaßt.

3. Verfahren nach Anspruch 1 oder Anspruch 2, worin das Kupfermetall in Pulverform in einer Menge von 5 bis 40 Gew.-%, insbesondere 15 bis 20 Gew.-.%, zugegen ist, bezogen auf das Gewicht des anorganischen Trägers und der Aktivkomponente.

4. Verfahren nach einem der Ansprüche 1 bis 3, **dadurch gekennzeichnet, daß** die katalytische Hydrierung als Festbettreaktion in Rieselfahrweise oder in Sumpffahrweise durchgeführt wird.

5. Verfahren nach einem der Ansprüche 1 bis 3, **dadurch gekennzeichnet, daß** das Verfahren in Kreislauffahrweise durchgeführt wird.

6. Verfabren nach einem der Ansprüche 1 bis 5, **dadurch gekennzeichnet, daß** als Carbonylverbindung ein aliphatischer Aldehyd oder ein aliphatischer Hydroxyaldehyd oder ein Gemisch aus zwei oder mehr als zwei dieser Aldehyde verwendet wird.

7. Katalysator in Tablettenform, der einen anorganischen Träger, der TiO₂ enthält, und als Aktivkomponente Kupfer oder ein Gemisch aus Kupfer mit mindestens einem der Metalle, ausgewählt aus der Gruppe Zink, Aluminium, Cer, einem Edelmetall und einem Metall der VIII. Nebengruppe, umfaßt, worin der Katalysator zusätzlich Kupfermetall in Pulverform enthält und eine spezifische Kupferoberfläche von maximal 10 m²/g aufweist.

8. Katalysator nach Anspruch 7, worin das Trägermaterial eine Mischung aus TiO₂ und Al₂O₃ oder eine Mischung aus TiO₂ und ZrO₂ oder eine Mischung aus TiO₂ und Al₂O₃ und ZrO₂ umfaßt.

9. Katalysator nach Anspruch 7 oder Anspruch 8, worin das Kupfermetall in Pulverform in einer Menge von 5 bis 40 Gew.-%, insbesondere 15 bis 20 Gew.-.%, zugegen ist, bezogen auf das Gewicht des anorganischen Träger und der Aktivkomponente.

10. Verfahren zur Herstellung eines Katalysators gemäß einem der Ansprüche 7 bis 9 in Tablettenform, das das Mischen der Komponenten und die Tablettierung der Mischung umfaßt, worin das Katalysatormaterial unter Zusatz von Kupfermetall in Pulverform zu Tabletten verformt wird.

## Claims

1. A process for the catalytic hydrogenation of a carbonyl compound or of a mixture of two or more carbonyl compounds in the presence of a catalyst in tablet form which comprises an inorganic support containing TiO₂, and as active component copper or a mixture of copper and at least one of the metals selected from the group of zinc, aluminum, cerium, a noble metal and a group VIII metal, wherein the catalyst additionally comprises copper metal in powder form and the specific copper surface area does not exceed 10 m²/g.

2. A process as claimed in claim 1, wherein, the support material comprises a mixture of TiO₂ and Al₂O₃ or a mixture of TiO₂ and ZrO₂ or a mixture of TiO₂ and Al₂O₃ and ZrO₂.

3. A process as claimed in claim 1 or 2, wherein the copper metal in powder form is present in an amount of from 5 to 40% by weight, in particular 15 to 20% by weight, based on the weight of the inorganic support and of the active component.

4. A process as claimed in any of claims 1 to 3, wherein the catalytic hydrogenation is carried out as fixed bed reaction in a downflow procedure or in an upflow procedure.

5. A process as claimed in any of claims 1 to 3, wherein the process is carried out in a recycle procedure.

6. A process as claimed in any of claims 1 to 5, wherein the carbonyl compound used is an aliphatic aldehyde or an aliphatic hydroxy aldehyde or a mixture of two or more than two of these aldehydes.

7. A catalyst in tablet form which comprises an inorganic support which contains TiO₂, and as active component copper or a mixture of copper with at least one of the metals selected from the group of zinc, aluminum, cerium, a noble metal and a group VIII metal, wherein the catalyst additionally comprises copper metal in powder form and has a specific copper surface area which does not exceed 10 m²/g.

8. A catalyst as claimed in claim 7, wherein the support material comprises a mixture of TiO₂ and Al₂O₃ or a mixture of TiO₂ and ZrO₂ or a mixture of TiO₂ and Al₂O₃ and ZrO₂.

9. A catalyst as claimed in claim 7 or claim 8, in which the copper metal in powder form is present in an amount of from 5 to 40% by weight, in particular 15 to 20% by weight, based on the weight of the inorganic support and of the active component.

10. A process for preparing a catalyst as claimed in any of claims 7 to 9 in tablet form, which comprises mixing the components and tableting the mixture, in which the catalyst material is shaped to tablets with addition of copper metal in powder form.

## Revendications

1. Procédé d'hydrogénation catalytique d'un composé carbonyle ou d'un mélange de deux ou de plusieurs composés carbonyle en présence d'un catalyseur sous forme de pastilles contenant un support inorganique, TiO₂, et comprenant, en tant que composant actif, du cuivre ou un mélange de cuivre et d'au moins un des métaux choisis dans le groupe formé par le zinc, l'aluminium, Cer, un métal noble et un métal du sous-groupe VIII, dans lequel le catalyseur comprend en outre du cuivre métallique en poudre et la surface spécifique du cuivre s'élève au maximum à 10 m²/g.

2. Procédé selon la revendication 1, **caractérisé en ce que** la matière support comprend un mélange de TiO₂ et Al₂O₃, ou un mélange de TiO₂ et ZrO₂, ou un mélange de TiO₂ et Al₂O₃ et ZrO₂.

3. Procédé selon la revendication 1 ou 2, dans lequel on ajoute du cuivre métallique en poudre à raison de 5% à 40% en poids, en particulier de 15% à 20% en poids, par rapport au poids du support inorganique et du composant actif.

4. Procédé selon l'une quelconque des revendications 1 à 3, **caractérisé en ce que** l'on effectue l'hydrogénation catalytique en réalisant une réaction en lit fixe, en régime de fond ou en régime de ruissellement.

5. Procédé selon l'une quelconque des revendications 1 à 3, caractérisé en ce l'on met en oeuvre le procédé dans des conditions de recyclage.

6. Procédé selon l'une quelconque des revendications 1 à 5, **caractérisé en ce que** l'on utilise en tant que composé carbonyle, un aldéhyde aliphatique ou un hydroxyaldéhyde aliphatique ou un mélange de deux ou de plusieurs de ces aldéhydes.

7. Catalyseur en forme de pastilles, contenant un support inorganique, TiO₂, et comprenant, en tant que composant actif, du cuivre ou un mélange de cuivre et d'au moins un des métaux choisis dans le groupe formé par le zinc, l'aluminium, Cer, un métal noble et un métal du sous-groupe VIII, dans lequel le catalyseur contient en outre du cuivre métallique en poudre et présente une surface spécifique du cuivre de 10 m²/g au maximum.

8. Catalyseur selon la revendication 7, dans lequel la matière support comprend un mélange de TiO₂ et Al₂O₃, ou un mélange de TiO₂ et ZrO₂, ou un mélange de TiO₂ et Al₂O₃ et ZrO₂.

9. Catalyseur selon la revendication 7 ou 8, dans lequel on ajoute du cuivre métallique en poudre à raison de 5% à 40% en poids, en particulier de 15% à 20% en poids, par rapport au poids du support inorganique et du composant actif.

10. Procédé pour préparer un catalyseur selon l'une quelconque des revendications 7 à 9 sous forme de pastilles, comprenant l'étape consistant à mélanger les composants et l'étape consistant à transformer le mélange en pastilles, dans lequel la matière de catalyseur est façonnée en forme de pastilles lors de l'addition du cuivre métallique en poudre est façonnée en forme de pastilles.
